## Europäisches Patentamt

## European Patent Office

## Office européen des brevets

(19)

(11) Veröffentlichungsnummer: **0 285 041**
**B1**

(12)

# EUROPÄISCHE PATENTSCHRIFT

(45) Veröffentlichungstag der Patentschrift:
**19.12.90**

(21) Anmeldenummer: **88104905.0**

(22) Anmeldetag: **26.03.88**

(51) Int. Cl.⁵: **B01J 8/38**, B01J 8/22
// C12M1/40

(54) Verfahren und Vorrichtung zum Betrieb von Wirbelschicht-Reaktoren.

(30) Priorität: **02.04.87 DE 3711177**

(43) Veröffentlichungstag der Anmeldung:
**05.10.88 Patentblatt 88/40**

(45) Bekanntmachung des Hinweises auf die Patenterteilung:
**19.12.90 Patentblatt 90/51**

(84) Benannte Vertragsstaaten:
**DE FR IT**

(56) Entgegenhaltungen:
**EP-A- 0 149 151**

**PATENT ABSTRACTS OF JAPAN, Band 3,
Nr. 133 (C-63), 7. November 1979, Seite 161 C 63; &
JP-A 54114474
FOOD TECHNOLOGY, Band 26, Nr. 12, Dezember 1972,
Seiten 23-30, Chicago, US; G.E. BROWN et al.:
"Centrifugal fluidized bed"**

(73) Patentinhaber: **DORNIER GMBH, Postfach 1420,
D-7990 Friedrichshafen 1(DE)**

(72) Erfinder: **Lork, Wolfram, Am Silberberg 16,
D-7758 Daisendorf(DE)**

(74) Vertreter: **Landsmann, Ralf, Dipl.-Ing., c/o DORNIER
GMBH Postfach 1420, D-7990 Friedrichshafen 1(DE)**

## Beschreibung

Die Erfindung betrifft ein Verfahren und eine Vorrichtung zum Betrieb von Wirbelschicht-Reaktoren unter Schwerelosigkeit, insbesondere im Bereich der Raumfahrt, die es ermöglichen, das Gewicht von in den Weltraum zu transportierenden Reaktoren, insbesondere Bio-Reaktoren, drastisch zu reduzieren und eine Schonung des Prozessmaterials gegenüber terrestrisch betriebenen Wirbeschicht-Bioreaktoren zu erreichen.

Wirbelschicht-Reaktoren (Gas/Feststoffsysteme) sind aus der Literatur für verschiedene Anwendungszwecke bekannt. Beispielsweise werden sie zur thermischen Materialbehandlung, wie Trocknen und Kühlen, in Cracking-Prozessen, bei der Kohleverbrennung, in der Zementindustrie und in der Biotechnologie eingesetzt. In allen Fällen wird ein Gasstrom oder eine wässrige Lösung von unten nach oben mit einer Lockerungsgeschwindigkeit in das Prozessmaterial geführt, so daß das Material während des Prozesses in einem Schwebezustand gehalten wird, während es vom Gas durchströmt wird. Die Lockerungsgeschwindigkeit ist die minimale Geschwindigkeit der kontinuierlich strömenden Phase, mit der der Schwebezustand erreicht wird.

Bei Bioreaktoren, die herkömmlich submers betrieben werden, das heißt das Prozessmaterial schwebt aufgrund seines spezifischen Gewichtes in einer wässrigen Lösung, hat der Übergang zu einem Wirbelschicht-System mit Gasströmung verschiedene Vorteile. Es wird eine höhere Substratkonzentration, ein besserer Gaseintrag und die unproblematischere Abfuhr der Prozesswärme und flüchtiger Prozessprodukte erreicht. Da Bioreaktion auch für verschiedene Anwendungen im Bereich der Raumfahrt diskutiert werden, erwähnenswert sind hier die Herstellung von Pharmazeutika und die biologischen Lebenserhaltungssysteme, spielt neben dem allgemeinen Wirkungsgrad auch das Gewicht solcher Systeme eine große Rolle.

Ein noch offenes Problem bei terrestrisch betriebenen Wirbelschicht-Bioreaktoren ist die Scher- und Abriebempfindlichkeit von biologischem Material, beispielsweise von Zellkulturen, die beim Durchmischen in Submerskulturen, aber auch bei Wirbelschicht-Kulturen unter Erdgravitationsbeschleunigung auftreten. Der Wirbelschicht-Reaktor verlangt einen Gasstrom, der groß genug ist, um über die entsprechenden Reibungskräfte am Prozessmaterial die Wirkung der Schwerkraft zu kompensieren. Diese Kräfte sind aber für empfindliche Zellen zu groß.

Bei einem Betrieb dieses Reaktortyps im Weltraum unter Schwerelosigkeit entfällt die dem Gasstrom entgegengerichtete Gravitationsbeschleunigung, die für die Aufrechterhaltung des Schwebezustandes erforderlich ist.

Aufgabe der Erfindung ist es, ein Verfahren und eine Vorrichtung zum Betreiben eines Wirbelschichtreaktors unter Schwerelosigkeit anzugeben.

Diese Aufgabe wird durch die Erfindung nach Anspruch 1 und den weiteren Ansprüchen gelöst.

Da bei einem Verfahren, das unter den Bedingungen der Schwerelosigkeit betrieben wird, die Gravitationsbeschleunigung, die hier wichtig für den Prozessablauf ist, entfällt, muß ein entsprechender Ersatz gefunden werden, der auch unter Schwerelosigkeit verfügbar ist. Ein solcher Ersatz ist die Radialbeschleunigung, die dem System durch den Betrieb des Reaktors auf einer Zentrifuge auferlegt wird. Dabei läßt sich der Zentrifugenantrieb und die Antriebsgeschwindigkeit frei variieren, wodurch auch die resultierende Zentrifugalkraft frei einstellbar ist. Sie kann somit einen optimalen Wert erhalten, der groß genug ist, um die Ver- und Entsorgung der prozessbeteiligten Größen zu gewährleisten, aber klein genug ist, um Schäden am Material zu vermeiden. Der hierbei angewandte Wirbelschicht-Reaktor verringert die Masse eines Algen- oder Bakterienreaktors herkömmlicher submerser Art auf weniger als ein Zwanzigstel, da statt der Versorgung mit einer Flüssigkeit wie wässrige Lösung nur noch feuchtes Gas wie angereicherte Luft benötigt wird.

Beim Verfahren gemäß der Erfindung halten sich die durch die Radialbeschleunigung und durch die Gasströmung bewirkten Kräfte $F_{rot}$ und $F_{flow}$ im Gleichgewicht. Dieses gilt aber nur in der eigentlichen Prozesszone. Um zufällige Fluktuationen zu stabilisieren, soll der Gasstrom so beschaffen sein, daß Partikel, die nach ausserhalb der Prozesszone gelangen, wieder in diese zurückgeführt werden. Um dieses zu erreichen, muß die Geometrie der Kammer entsprechend beschaffen sein:

Für die Prozesszone gilt:

$F_{rot} = m \cdot \omega^2 \cdot r$, also eine lineare Abhängigkeit der Zentrifugalkraft vom Abstand zum Drehpunkt.

$F_{flow} = 6 \cdot \pi \cdot \eta \cdot R \cdot v$ (Stoke) beschreibt die Reibungskraft eines Teilchens mit dem Gasstrom der Geschwindigkeit v. Diese Gasgeschwindigkeit ist umgekehrt proportional zur Querschnittsfläche der durchströmten Kammer : $v \sim 1/A$. Um Fluktuationen zu begegnen, soll in der Nähe des Drehpunktes die Zentrifugalkraft überwiegen (durch größeren Kammerquerschnitt wird die Gasgeschwindigkeit gesenkt), an der Aussenseite soll die Gasströmung überwiegen (kleiner Kammerquerschnitt).

Die Erfindung wird anhand von Figuren näher erläutert.

Die Figur 1 zeigt eine Funktionsskizze einer Zentrifuge in prinzipieller Darstellung.

Die Figur 2 zeigt eine Zentrifuge mit sechs Speichen.

Die Figur 3 zeigt eine besondere Ausgestaltung der Kammerform.

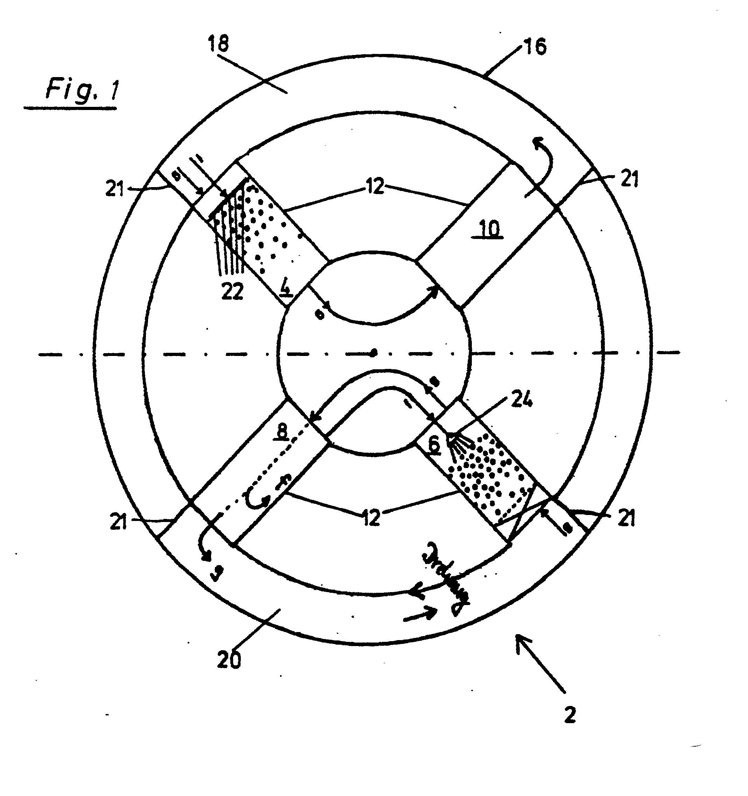

In der Figur 1 werden zwei Versionen dargestellt, die sich in der Zuführung der Flüssigphase unterscheiden. Dabei zeigt die Funktionsskizze ein Beispiel für die Realisierung einer Zentrifuge 2, bei der den eigentlichen Reaktorkammern 4 und 6 gewichtsmäßig die notwendigen Nebensysteme, Kondensationskammer 8 und Feuchtigkeitsregler 10, gegenüber angeordnet sind. Dies hat den Vorteil, auch dort Verfahren zu verwenden, die bei terrestrischer Anwendung die Schwerkraft nutzen, beispielsweise der Abfluß von Kondensationswasser. Dazu sind die notwendigen Aggregate beispielsweise in Speichen 12 um eine Achse 14 herum angeordnet, wobei die Zahl der Speichen nicht auf vier beschränkt ist.

Die Speichen 12 tragen einen Ring 16, der hohl und in vier Segmente unterteilt ist, von denen Segment 18 und Segment 20 der Gaszufuhr dienen und durch Trennstücke 21 von den übrigen Segmenten getrennt sind.

Die Figur 1 stellt zwei unterschiedliche Versionen der Flüssigkeits- und Gaszuführung (f bzw. g) mit Gas als kontinuierlicher Phase dar. In beiden Versionen strömt das Gas g von unten nach oben, bezogen auf die Kammern, in die Partikelschicht in den Reaktorkammern 4 und 6.

In der in der Figur 1 oben dargestellten Version, wird die Flüssigkeitsphase f durch Düsen 22 von unten zugeführt, in der in der Figur unteren Version durch Düsen 24 von oben. Um die Partikel in den Reaktorkammern in der Schwebe zu halten, wirkt die Zentrifugalkraft, durch die Rotation der Zentrifuge in angegebener Drehrichtung, dem Druck des Gases g entgegen.

In den beiden Versionen wird die zugeführte flüssige Phase, beispielsweise aus wässriger Lösung, vollständig in Form von Wasserdampf mit dem Gasstrom abgeführt. In der in der Figur oberen Version wird das Gasgemisch dann über einen Feuchtigkeitsregler 10 wieder durch das Segment 18 dem Reaktorraum 4 zugeführt. Im Feuchtigkeitsregler 10 wird dazu die Feuchte des Gasgemisches abgereichert und das Gas durch das Segment 18 in Richtung untere Gaszuführung der Reaktorkammer 4 geleitet.

In der in der Figur unteren Version hingegen gelangt das Gasgemisch in die Kondensationskammer 8, wo das Gemisch kondensiert. Die Flüssigphase f strömt zu den Düsen 24 zurück und das Gas g durch das Segment 20 wieder in Richtung untere Gaszuführung der Reaktorkammer 6.

Die Hilfseinrichtungen zur Gas- und Flüssigkeitszuführung, die über das Dargestellte hinausgehen, und ein Flüssigkeitsreservoir sind in dieser prinzipiellen Darstellung nicht gezeigt.

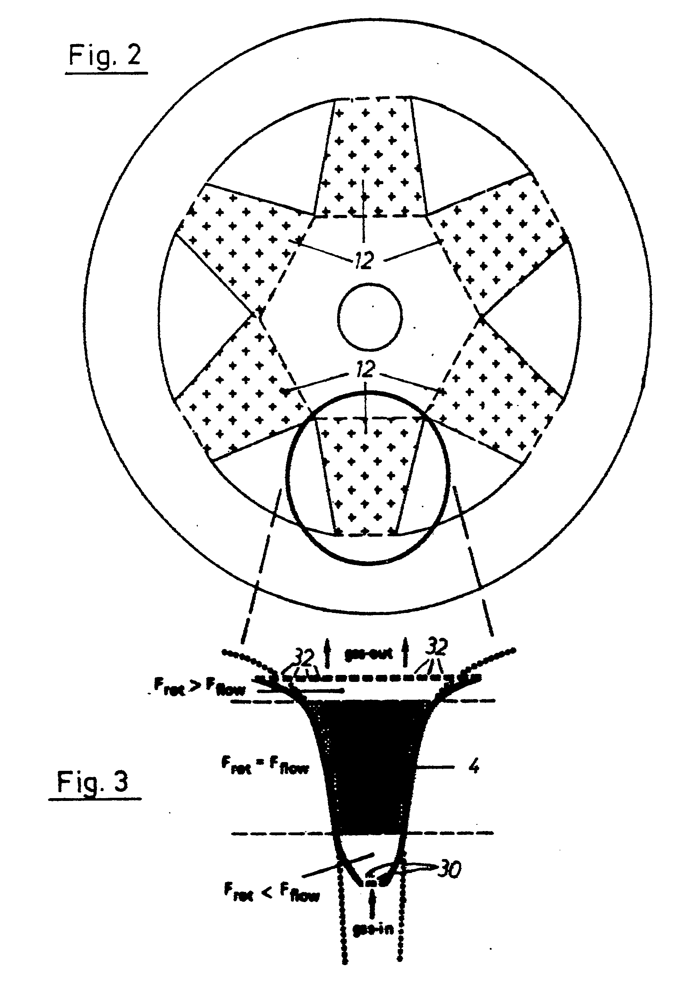

Figur 2 zeigt eine erfindungsgemäße Zentrifuge 2 im Querschnitt mit sechs Speichen 12. Die Querschnittsfläche der Speichen 12, und damit die Querschnittsfläche der sich in den Speichen 12 befindlichen Bauteile Kammern 4, 6 oder Nebenaggregate 8, 10, nimmt zum Rotationszentrum hin mit 1/r zu, um die mit dem Abstand vom Drehpunkt zunehmende Zentrifugalkraft kompensieren zu können. Dies führt zur vorteilhaften Ausgestaltung der Kammern in Hyperbelform im Prozessbereich. Dies ist in Figur 3 gezeigt.

Gaseinlaßschlitze 30 und Gasauslaßschlitze 32 sind gezeigt, die das Zellmaterial in den Kammern halten. Im Mittelbereich der Kammer heben sich Radialbeschleunigung und durch Gasströmung bewirkte Kräfte auf.

## Patentansprüche

1. Verfahren zum Betrieb von Wirbelschicht-Reaktoren in Schwerelosigkeit, insbesondere im Weltraum, **dadurch gekennzeichnet**, daß die Gravitationsbeschleunigung durch eine Radialbeschleunigung ersetzt wird.

2. Verfahren nach Anspruch 1, dadurch gekennzeichnet, daß Prozessmaterial durch die Radialbeschleunigung gegen eine Gas- und/oder Flüssigkeitsströmung in einem Schwebezustand gehalten wird.

3. Verfahren nach den Ansprüchen 1 und 2, dadurch gekennzeichnet, daß die Radialbeschleunigung in der Schwerelosigkeit geringer eingestellt wird als die Erdgravitationsbeschleunigung.

4. Verfahren nach einem der Ansprüche 1 bis 3, dadurch gekennzeichnet, daß der Wirbelschicht-Reaktor vorzugsweise mit Feuchtgas betrieben wird.

5. Verfahren nach Anspruch 4, dadurch gekennzeichnet, daß als Feuchtgas angereicherter Wasserdampf oder angereicherte Luft verwendet wird.

6. Verfahren nach einem der Ansprüche 1 bis 5, dadurch gekennzeichnet, daß in eine Reaktorkammer Flüssigkeit zugeführt wird.

7. Verfahren nach Anspruch 6, dadurch gekennzeichnet, daß die Flüssigkeit eine wässrige Lösung ist.

8. Verfahren nach einem der vorherigen Ansprüche, dadurch gekennzeichnet, daß die Flüssigkeit über Düsen zugeführt wird.

9. Vorrichtung zur Durchführung des Verfahrens nach dem Oberbegriff des Anspruchs 1, dadurch gekennzeichnet, daß in den Speichen einer radförmigen Zentrifuge Reaktorkammern (4, 6) und Nebenaggregate (8, 10) angeordnet sind.

10. Vorrichtung nach Anspruch 9, dadurch gekennzeichnet, daß an den Reaktorkammeroberseiten oder -unterseiten Düsen (22, 24) angeordnet sind.

11. Vorrichtung nach Anspruch 9 oder 10, dadurch gekennzeichnet, daß die Querschnittsfläche der Reaktorkammern (4, 6) mit zunehmendem Radius der Zentrifuge (2) abnimmt.

12. Vorrichtung nach Anspruch 11, dadurch gekennzeichnet, daß die Reaktorkammern (4, 6) hyperbolisch ausgebildet sind.

## Claims

1. Process for operating fluidized reactors in zero-gravity, in particular in space, characterised in that the gravitational acceleration is replaced by a radial acceleration.

2. Process as claimed in claim 1, characterised in that processing material is held in an oscillating state against a gas and/or fluid current by means of radial acceleration.

3. Process according to claims 1 and 2, characterised in that the radial acceleration in zero-gravity is set lower than the earth gravitational acceleration.

4. Process according to one of claims 1 to 3, characterised in that the fluidized reactor is preferably operated by means of humid gas.

5. Process according to claim 4, characterised in that enriched water vapour or enriched air is used as the humid gas.

6. Process according to one of claims 1 to 5, characterised in that a fluid is delivered into a reactor chamber.

7. Process according to claim 6, characterised in that the fluid is an aqueous solution.

8. Process according to one of the preceding claims, characterised in that the fluid is delivered via nozzles.

9. Apparatus for performing the process according to the preamble of claim 1, characterised in that reactor chambers 4, 6 and auxiliary units 8, 10 are disposed in the spokes of a wheel-shaped centrifuge.

10. Apparatus according to claim 9, characterised in that nozzles 22, 24 are disposed on the upper or lower sides of the reactor chambers.

11. Apparatus according to claim 9 or 10, characterised in that the cross-sectional area of the reactor chambers 4, 6 decreases as the radius of the centrifuge 2 increases.

12. Apparatus according to claim 11, characterised in that the reactor chambers 4, 6 are hyperbolic.

## Revendications

1. Procédé pour faire fonctionner des réacteurs à lit fluidisé en apesanteur, en particulier dans l'espace, caractérisé en ce que l'accélération gravitationnelle est remplacée par une accélération radiale.

2. Procédé selon la revendication 1, caractérisé en ce que le matériau traité est maintenu par l'accélération radiale contre un courant de gaz et/ou de liquide dans un état de flottement.

3. Procédé selon les revendications 1 et 2, caractérisé en ce que l'accélération radiale dans l'état d'apesanteur est réglée plus faible que l'accélération gravitationnelle terrestre.

4. Procédé selon l'une des revendications 1 à 3, caractérisé en ce que le réacteur à lit fluidisé fonctionne de préférence avec du gaz humide.

5. Procédé selon la revendication 4, caractérisé en ce qu'il utilise comme gaz humide de la vapeur d'eau enrichie ou de l'air enrichi.

6. Procédé selon l'une des revendications 1 à 5, caractérisé en ce que du liquide est amené dans une chambre du réacteur.

7. Procédé selon la revendication 6, caractérisé en ce que le liquide est une solution aqueuse.

8. Procédé selon l'une des revendications précédentes, caractérisé en ce que le liquide est amené par l'intermédiaire de buses.

9. Dispositif pour la mise en oeuvre du procédé selon le préambule de la revendication 1, caractérisé en ce que dans les rayons d'une centrifugeuse en forme de roue sont disposés des chambres de réacteur (4, 6) et des dispositifs secondaires (8, 10).

10. Dispositif selon la revendication 3, caractérisé en ce que des buses (22, 24) sont disposées sur les surfaces supérieures ou inférieures des chambres de réacteur.

11. Dispositif selon l'une des revendications 9 ou 10, caractérisé en ce que l'aire de la section transversale des chambres de réacteur (4, 6) diminue avec l'augmentation du rayon de la centrifugeuse (2).

12. Dispositif selon la revendication 11, caractérisé en ce que les chambres de réacteur (4, 6) présentent une forme hyperbolique.

*Fig. 1*

Fig. 2

Fig. 3